# EUROPÄISCHE PATENTANMELDUNG

(11) **EP 1 269 909 A1**
(43) Veröffentlichungstag der Anmeldung: **02.01.2003**
(21) Anmeldenummer: 02013787.3
(22) Anmeldetag: 21.06.2002
(51) Int. Cl.: A61B 5/00

(54) **Verfahren und eine Vorrichtung zum Erkennen von krankhaften Veränderungen an einer Gewebeoberfläche, insbesondere Zähnen**

(30) Priorität: 22.06.2001 DE 10129694
(71) Anmelder: Sirona Dental Systems GmbH, 64625 Bensheim (DE)
(72) Erfinder: Zanger, Ulf, 76356 Weingarten (DE); Behringer, Wolfgang, 64625 Bensheim (DE)
(74) Vertreter: Sommer, Peter

(57) **Zusammenfassung**

Verfahren und eine Vorrichtung zum Erkennen von krankhaften Veränderungen wie Karies, Plaque, Konkrementen oder bakteriellem Befall an einer Gewebeoberfläche, insbesondere an Zähnen

Die Erfindung betrifft Verfahren und eine Vorrichtung zum Erkennen von krankhaften Veränderungen wie Karies, Plaque, Konkrementen oder bakteriellem Befall an einer Gewebeoberfläche, insbesondere an Zähnen unter Verwendung einer durch eine Anregungsstrahlung hervorgerufenen Fluoreszenzstrahlung, welche im Falle des Auftretens einer krankhaften Veränderung in einem Teilbereich ein Strahlungsspektrum mit gegenüber der gesunden Gewebeoberfläche erhöhter Fluoreszenzstrahlung aufweist.

Zusätzlich erfolgt eine Erfassung und Auswertung der von der Gewebeoberfläche rückgestreuten Strahlung in mindestens einem Teilbereich des Strahlungsspektrums der Anregungsstrahlung, wobei der Teilbereich der ausgewerteten rückgestreuten Anregungsstrahlung und der Teilbereich der ausgewerteten Fluoreszenzstrahlung zumindest bezüglich der zu erwartenden Maximalwerte überschneidungsfrei sind.

## Beschreibung

### Technisches Gebiet

Die Erfindung betrifft ein Verfahren und eine Vorrichtung zum Erkennen von krankhaften Veränderungen wie Karies, Plaque, Konkrementen oder bakteriellem Befall an einer Gewebeoberfläche, insbesondere an Zähnen.

Bei Beleuchtung von Gewebe mit Licht aus dem violetten und blauen Spektralbereich senden biologische Gewebe eine Fluoreszenzstrahlung aus. Die Art der Fluoreszenz d. h. ihr Spektrum gibt Aufschluß über die Zusammensetzung des Gewebes. Gleiches gilt für Bakterien, welche wiederum aus biologischem Gewebe bestehen, und deren Stoffwechselprodukte. Bei der Entstehung von Plaque und Konkrementen werden die Stoffwechselprodukte in die Ablagerungen auf den Zahnoberflächen eingelagert und somit mittels Fluoreszenz detektierbar. Diese Ablagerungen sind mit verantwortlich für die Entstehung von parodontalen Erkrankungen. Da sie sich aufgrund der engen räumlichen Verhältnisse zwischen Zahn und Zahnfleisch einer visuellen Beurteilung weitestgehend entziehen, ist es, ohne die Zahnfleischtasche zu öffnen, schwierig zu entscheiden, ob Auflagerungen am Zahn vorhanden sind oder nicht. Insbesondere ist es unmöglich zu erkennen, ob durch eine Behandlung die Auflagerungen vollständig entfernt wurden oder ob schädliche Reste auf der Zahnoberfläche zurückblieben. Der Einsatz eines Fluoreszenzverfahrens beschränkt sich jedoch nicht auf das Gebiet der parodontalen Erkrankungen. Vielmehr ist der Einsatz auch bei anderen Erkrankungen der Mundhöhle möglich. Dies sind beispielhaft Karies, bakterielle Beläge auf Zunge, Gaumen und Zahnfleisch sowie Krebs und dessen Vorstufen.

### Stand der Technik

Bisher bekannte Verfahren und Vorrichtungen zur Diagnostik an Zähnen mit Licht, wie beispielsweise in EP 0 862 896 A2, DE 42 00 741 und DE 198 25 021 A1 beschrieben, weisen mehrere Nachteile beziehungsweise Beschränkungen auf.

Bei Änderung der optischen Eigenschaften des Handstückes, wie Lichtübertragung und Lichtaus- und Einkopplung, verändert sich der angezeigte Messwert und es kann zu einer falschen Beurteilung des untersuchten Zahnbereiches kommen. Die Änderungen der optischen Eigenschaften können sich durch Alterung des Handstückes, Streuungen der Parameter gleichartiger Handstücke und Einsatz unterschiedlicher Handstücke ergeben. Bei den vorgenannten Verfahren ist es daher nötig, die Vorrichtung unmittelbar vor der Untersuchung in geeigneter Weise zu kalibrieren.

Die beschriebenen Handstücke strahlen am distalen Ende das Licht im wesentlichen in axialer Richtung ab. Der Empfang des vom Zahn abgegebenen Lichtes erfolgt ebenfalls im wesentlichen in axialer Richtung des Handstückes. Mit einem solchen Handstück sind enge Bereiche in der Mundhöhle, wie beispielsweise die Zahnzwischenräume nicht oder nur schwierig zu erreichen. Insbesondere ist es mit derartigen Handstücken nicht möglich eine Untersuchung auf Konkremente in parodontalen Taschen, also zwischen Zahn und Zahnfleisch, durchzuführen.

Entzündete parodontale Taschen bluten meist. Blut absorbiert Licht in einigen Teilen des sichtbaren Spektrums, insbesondere im blauen und grünen Wellenlängenbereich, sehr stark. Dies führt dazu, daß auf dem Weg des Lichtes vom Handstück zum Zahn und wieder zurück ein erheblicher Anteil des Lichtes absorbiert wird. Dies macht eine Befundung oft unmöglich.

Diese Probleme sollen ganz oder zumindest teilweise gelöst werden.

### Darstellung der Erfindung

Das erfindungsgemäße Verfahren zum Erkennen von krankhaften Veränderungen wie Karies, Plaque, Konkrementen oder bakteriellem Befall an einer Gewebeoberfläche, insbesondere an Zähnen, umfasst die Schritte:
a) Bestrahlen einer zu untersuchenden Gewebeoberfläche mit einer Anregungsstrahlung mit einem ersten Strahlungsspektrum, die an der Gewebeoberfläche eine Fluoreszenzstrahlung mit einem zweiten Strahlungsspektrum hervorruft, und
b) Erfassen und Auswerten des Strahlungsspektrums der an der Gewebeoberfläche (16) durch die Anregungsstrahlung hervorgerufenen Fluoreszenzstrahlung zumindest in einem Teilbereich mit einer im Falle des Auftretens einer krankhaften Veränderung wie Karies, Plaque, Konkremente oder bakterieller Befall gegenüber der gesunden Gewebeoberfläche erhöhter Fluoreszenzstrahlung.

Dadurch, dass zusätzlich eine Erfassung und Auswertung der von der Gewebeoberfläche rückgestreuten Strahlung in mindestens einem Teilbereich des Strahlungsspektrums der Anregungsstrahlung erfolgt, wobei der Teilbereich der ausgewerteten rückgestreuten Anregungsstrahlung und der Teilbereich der ausgewerteten Fluoreszenzstrahlung zumindest bezüglich der zu erwartenden Maximalwerte überschneidungsfrei sind, lässt sich die Diagnosefähigkeit der gemessenen Signale bei krankhaften Veränderungen entscheidend verbessern.

Gemäß einer Weiterbildung erfolgt nach der Auswertung des von der Gewebeoberfläche rückgestreuten Teilbereichs der Anregungsstrahlung eine Anpassung (Normierung) des an der Gewebeoberfläche durch die Anregungsstrahlung hervorgerufenen Spektrums der Fluoreszenzstrahlung, indem aus der ausgewerteten rückgestreuten Anregungsstrahlung durch Vergleich des Spektrums mit einem vorgegebenen Spektrum ein Abbildungsfaktor erzeugt wird, der auf das Spektrum der Fluoreszenzstrahlung angewandt wird.

Dies hat den Vorteil, dass unter Berücksichtigung des individuellen Gerätezustandes und der Präparationsbedingungen ein auswertbares Signal mit hinreichendem Abstand zu einem vorgegebenen standardisierten Vergleichswert bereitgestellt werden kann. In einer Weiterbildung ist durch Vergleichen des mit dem Abbildungsfaktor abgebildete Wertes des Spektrums der Fluoreszenzstrahlung mit einem vorgegebenen Standardwert eine Abweichung feststellbar, welche eine Diagnose ermöglicht.

Vorteilhafterweise sind die Spektren jeweils als schmalbandiger Bereich ausgewählt, so dass einerseits die ausgewerteten Bereiche des Spektrums der Reflexionsstrahlung und der Fluoreszenzstrahlung ohne wesentlichen Einfluss aufeinander sind und andererseits kostengünstige Empfänger verwendet werden können.

Vorteilhafterweise wird zumindest ein Teil des reflektierten Teils der Anregungsstrahlung in einem ersten Empfänger ausgewertet, wobei die vom Zahn ausgehende Strahlung vor dem Auftreffen auf einen zweiten Empfänger so gefiltert wird, dass im zweiten Empfänger im wesentlichen nur der diagnostisch relevante Teil der Fluoreszenzstrahlung und nicht die Anregungsstrahlung erfasst wird.

Die Anregung der zu untersuchenden Oberfläche erfolgt dabei vorzugsweise mit Licht eines Teilbereiches des Wellenlängenbereichs von 380nm - 490nm, wobei die Auswertung in mindestens zwei überschneidungsfreien Teilbereichen des Wellenlängenbereichs von 380nm - 750nm erfolgt und wobei einer der Teilbereiche für die Auswertung der Wellenlängenbereich der Anregungsstrahlung oder ein Teilbereich hiervon ist, beispielsweise eine Wellenlänge von 465 nm.

Vorteilhafterweise wird als Strahlung Licht und als Messgröße die Lichtintensität zu dem jeweiligen Be-reich der Wellenlänge verwendet.

Ein weiterer Gegenstand der Erfindung ist eine Vorrichtung zum Erkennen von krankhaften Veränderungen wie Karies, Plaque, Konkrementen oder bakteriellem Befall an einer Gewebeoberfläche, insbesondere an Zähnen, mit einer Einrichtung zur Erzeugung einer Anregungsstrahlung, welche mit Hilfe eines zahnärztlichen Handstücks auf eine zu untersuchende Gewebeoberfläche gerichtet ist, und mit Mitteln zur Übertragung von aus einer Quelle austretender Anregungsstrahlung zu der zu bestrahlenden Gewebeoberfläche und Mitteln zum Erfassen einer an der bestrahlten Gewebeoberfläche angeregten Fluoreszenzstrahlung, wobei weiterhin Mittel zum Erfassen einer an der bestrahlten Gewebeoberfläche mit der Wellenlänge der Anregungsstrahlung rückgestreuten Reflexionsstrahlung vorgesehen sind.

Vorteilhafterweise erfolgt die Anregung der zu untersuchenden Oberfläche mit Strahlung eines ersten Teilbereiches des Strahlungsspektrums, wobei über Mittel die Erfassung der von der Gewebeoberfläche ausgehenden Strahlung in mindestens zwei überschneidungsfreien Teilbereichen des Strahlungsspektrums erfolgt und wobei einer der Teilbereiche für die Auswertung der Teilbereich der Anregungsstrahlung oder ein weiter eingeschränkter Teilbereich hiervon ist.

Gemäß einer Weiterbildung ist im Strahlengang der Anregungsstrahlung ein Filter vorgesehen, welches nur einen geeigneten Teilbereich der von der Quelle ausgehenden Strahlung durchlässt.

Gemäß einer Weiterbildung ist im Strahlengang der Reflexionsstrahlung ein Filter vorgesehen, um die relativ starke rückgestreute Anregungsstrahlung abzuschwächen und/oder auf einen Teilbereich zu beschränken.

Gemäß einer Weiterbildung ist im Strahlengang der Fluoreszenzstrahlung ein Filter vorgesehen, um diese auf einen Teilbereich zu beschränken.

Gemäß einer Weiterbildung ist ein Lichtleiter zur Auskopplung von Reflexionsstrahlung zu einem ersten optischen Empfänger, der das Licht in ein elektrisches Signal umwandelt, und ein weiterer Lichtleiter, der zu einem zweiten optischen Empfänger für eine Auswertung der Fluoreszenzstrahlung innerhalb eines vorgesehenen Spektralbereichs führt, vorgesehen.

Gemäß einer Weiterbildung ist ein Speicherbereich für einen abgelegten Standardwert der Anregungsstrahlung oder eines Teilbereichs davon als Vergleichsgröße vorgesehen und sind Vergleichsmittel des Standardwerts mit dem nach der Auswertung der vom Zahn reflektierten Anregungsstrahlung oder des Teilbereichs vorhanden sowie Korrekturmittel zur Anpassung (Normierung) des am Zahn durch die Anregungsstrahlung hervorgerufenen Teilbereichs der Fluoreszenzstrahlung. Diese können Teil einer Auswerteeinheit sein. Damit ist eine messungsspezifische Korrektur der Messwerte und ein Vergleich mit dem vorgegebenen vorzugsweise normierten Standardwert möglich.

Gemäß einer Weiterbildung ist die Lichtquelle eine gepulste oder modulierte Leuchtdiode oder ein Laserdiode. Dadurch wird eine Trennung der auszuwertenden Strahlung von der Umgebungsstrahlung mit im Stand der Technik bekannten Maßnahmen möglich. Dabei kann die Lichtquelle auch gefiltert sein, um eine Überschneidung mit dem Diagnosebereich auszuschließen.

Gemäß einer Weiterbildung sendet und empfängt das Handstück Strahlung, insbesondere Licht aus mindestens einer seitlichen Richtung, bevorzugt aus einer oder zwei Richtungen unter etwa 90 Grad zu seiner Längsachse. Durch den seitlichen Austritt und Eintritt ist das Arbeiten in Zahnfleischtaschen möglich.

Vorteilhafterweise erfolgt die Ablenkung des Lichtes durch Reflexion an einer schräggestellten Oberfläche.

Gemäß einer Weiterbildung erfolgt die Ablenkung des Lichtes durch Reflexion an zwei entgegengesetzt abstrahlenden Oberflächen.

Gemäß einer Weiterbildung besteht der Lichtleiter aus einer einzelnen Faser, welche für Hin- und Rückleitung des Lichtes benutzt wird. Alternativ dazu ist es möglich, dass der Lichtleiter aus einem Faserbündel besteht.

Vorteilhafterweise weist das Handstück der erfindungsgemäßen Vorrichtung einen Kanal auf, durch welchen Spülflüssigkeit zugeführt und damit das Blut aus der Zahnfleischtasche gespült werden kann.

Wird dem Spülmedium eine bakterizide Substanz, wie beispielsweise Chlorhexidin, beigegeben, so ist gleichzeitig eine die Heilung der Parodontitis unterstützende Therapie möglich.

Erhält das distale Ende des Handstückes darüber hinaus zusätzlich Markierungen, welche die Eindringtiefe in die Tasche anzeigen, so sind in einem Arbeitsgang und mit dem gleichen Gerät die Suche nach Konkrementen bzw. Plaque, Beurteilung der Taschentiefe und eine bakterizide Therapie möglich.

Vorteilhafterweise ist eine optische, akustische oder sonstige Anzeige für die Überschreitung eines krankhafte Veränderungen bedeutenden vorgegebenen Schwellenwertes durch einen aus der gemessenen Strahlung zu der erwarteten Strahlung des gesunden Gewebes bestimmten Vergleichswertes vorgesehen.

Vorteilhafterweise ist der Schwellenwert einstellbar, so dass eine Nachjustierung der Anzeige durch den Benutzer möglich ist.

### Kurzbeschreibung der Zeichnung

In der Zeichnung sind Ausführungsbeispiele der Erfindung dargestellt. Es zeigt
- Fig. 1: das prinzipielle Blockschaltbild eines erfindungsgemäßen Fluoreszenzsystems;
- Fig. 2: zwei gemessene Spektren an einem Zahn;
- Fig. 3: die beiden normierten Kurven 19, 20 der Spektren aus Fig. 2;
- Fig. 4: die Einspeisung und die Auskopplung des Lichtes in ein Lichtleitkabel;
- Fig. 5: eine weitere Ausführungsform;
- Fig. 6: ein Handstück mit Lichtleiter;
- Fig. 7: eine Ausführungsform des distalen Endes 24 des Handstücks aus Fig. 6 im Detail;
- Fig. 8: einen Schnitt des distalen Endes entlang der Linie A aus Fig. 7.

### Ausführungsbeispiele der Erfindung

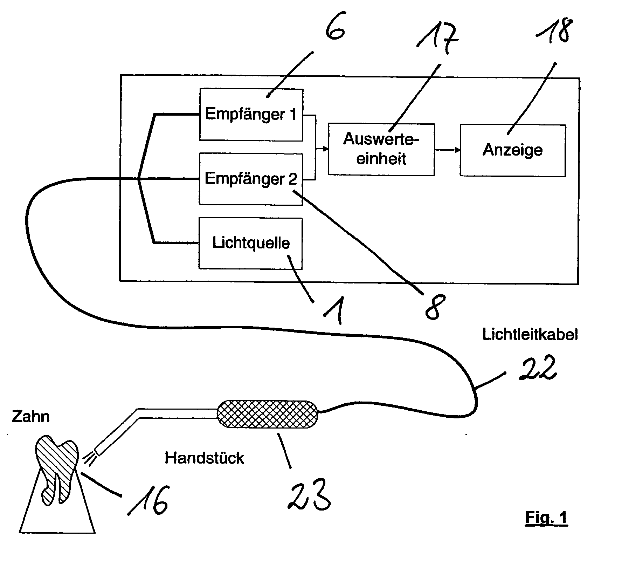

Fig.1 zeigt das prinzipielle Blockschaltbild eines erfindungsgemäßen Fluoreszenzsystems. Licht aus einer Lichtquelle 1 eines Gerätes wird in ein Lichtleitkabel eingekoppelt und von diesem zu einem speziell gestalteten Handstück 23 transportiert. Das dort distal austretende Licht beleuchtet die zu untersuchende Gewebeoberfläche, dargestellt durch einen Zahn 16. Das von dem Zahn 16 generierte Fluoreszenzlicht wird wiederum vom distalen Ende aufgesammelt und über das Lichtleitkabel 22 zum Gerät zurücktransportiert. Dieses enthält mindestens zwei Empfangskanäle 6, 8, welche unterschiedliche Bereiche der Wellenlänge empfangen und in elektrische Signale umwandeln. Die nachgeschaltete Auswerteeinheit 17 verarbeitet diese Signale und generiert ein Resultat, welches auf einer Anzeige 18 wahrnehmbar gemacht wird. Die Anzeige 18 gestattet dem Anwender eine Entscheidung bezüglich der Frage, ob die untersuchte Gewebeoberfläche krankhaft befallen ist oder nicht.

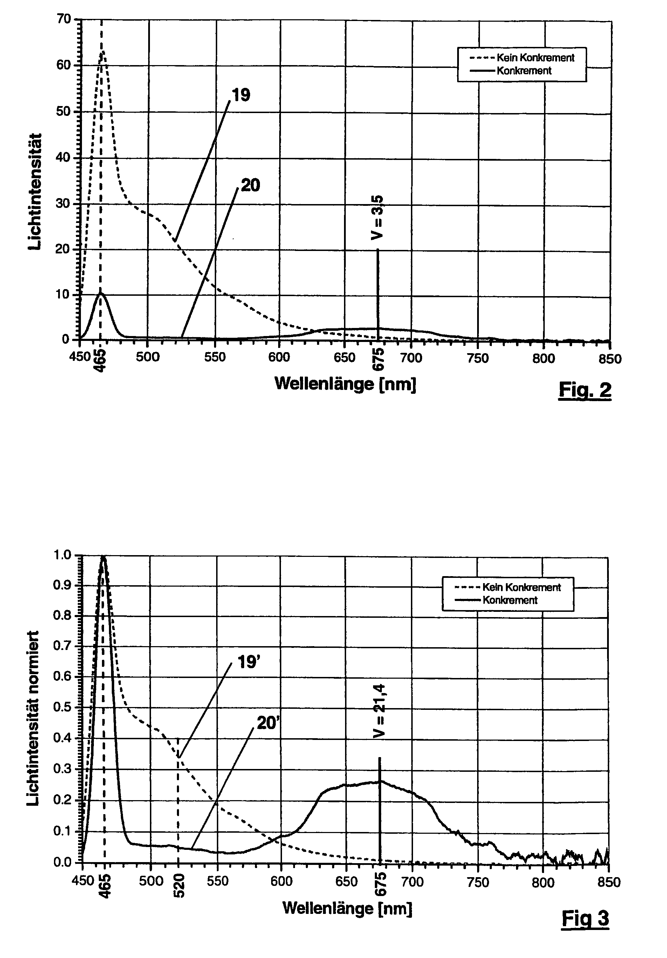

Fig. 2 zeigt beispielhaft zwei gemessene Spektren 19, 20 an einem Zahn. Die Anregung der Fluoreszenz erfolgte mit breitbandigem Licht im Bereich von 390-470 nm, wobei bei 406 nm ein Maximum der Anregung zur Fluoreszenz des Zahnes liegt. Bei etwa 465 nm ist ein Rest des reflektierten Anregungslichtes in beiden Kurven 19, 20 zu erkennen. Da die Intensität der Anregung etwa 1000-mal höher als die der Fluoreszenz ist, wurde das reflektierte Licht mit einem Filter entsprechend abgeschwächt. Es ist zu erkennen, daß sich die Kurve 20 der Messung am Konkrement deutlich von der Kurve 19 an einem sauberen Bereiches des Zahns unterscheidet. Das Konkrement zeigt im roten Spektralbereich um 675nm eine etwa 3,5-fach größere Lichtintensität wie die saubere Zahnoberfläche.

Die empfangene Lichtintensität ist nicht ausschließlich von dem Material der untersuchten Oberfläche abhängig.

Sie wird außerdem vom Abstand des Handstückes zur Zahnoberfläche und der Geometrie der Zahnoberfläche beeinflusst. Darüber hinaus ist die Lichtintensität stark von der Lichtübertragung durch das Handstück und das Lichtleitkabel abhängig. Ändern sich die optischen Eigenschaften dieser Teile, z. B. durch Alterung, oder wird das Handstück gegen ein anderes gleichartiges oder in der Bauart verschiedenes ausgetauscht, so entstehen hierdurch erhebliche Änderungen in den empfangenen Intensitäten. Dies macht es in der Praxis schwer oder unmöglich, für die empfangene Fluoreszenz Schwellwerte festzulegen, anhand derer eine Entscheidung zwischen Konkrement vorhanden bzw. kein Konkrement vorhanden zuverlässig getroffen werden kann.

Bekannte Systeme verwenden daher eine Messung an einem Prüfkörper bekannter Eigenschaften zur Kalibrierung des Gerätes. Dieser Vorgang ist jeweils vor den eigentlichen Messungen am Zahn oder im Mundraum durchzuführen.

Wird beispielsweise in einem zweiten Empfängerkanal zusätzlich ein Teil des rückgestreuten Anregungslichtes empfangen und ausgewertet, so kann damit bei jeder Messung am Zahn eine automatische Kalibrierung erfolgen.

Hierzu wird die Amplitude des Spektrums der Reflexionsstrahlung im Wellenlängenbereich der Anregungsstrahlung auf einen Normwert abgebildet, wodurch ein Korrekturwert ermittelt wird. Eine derartige Rechenoperation wird als Normierung bezeichnet, wenn der Normwert selbst 1 beträgt.

Da die in der Beschreibungseinleitung genannten Fehlerquellen sich auf die Amplituden über den ganzen Wellenlängenbereich in etwa gleich auswirken, also auf Fluoreszenz ebenso wie auf Reflexion, wird hierdurch ihr Einfluss herausgerechnet und damit Fehlerquellen beseitigt. Eine diagnostische Entscheidung kann daher mit einer erheblich höheren Zuverlässigkeit getroffen werden.

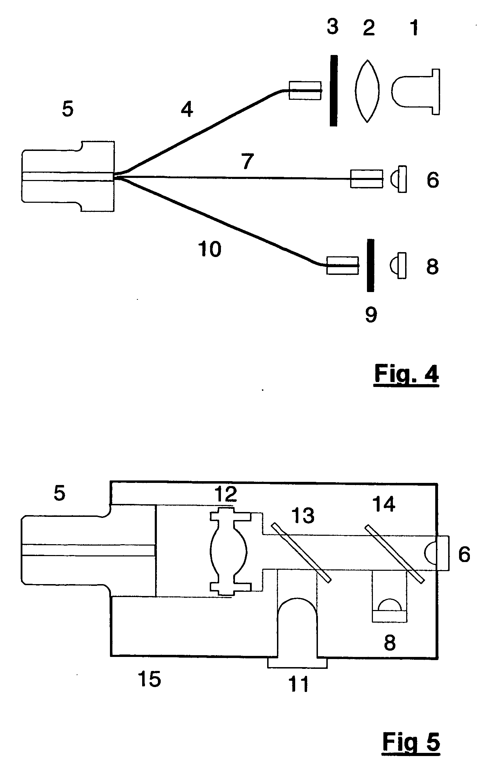

In Fig. 3 wurden die beiden Kurven 19, 20 der Einzelspektren aus Fig. 2 normiert, indem jeweils ihre Amplitude der Reflexion bei einer Wellenlänge von etwa 465 nm durch Multiplikation mit einem jeweils zur Kurve 19 bzw. 20 gehörenden Korrekturfaktor auf 1 gesetzt wurde und die Amplituden der übrigen Wellenlängen mit dem jeweils zugehörigen Korrekturfaktor multipliziert wurden, so dass die Kurven 19', 20' entstehen. Aufgrund der Normierung weisen beide Kurven bei einer Wellenlänge von 465 nm einen Wert von 1 auf. Der Korrekturfaktor für die Kurve 19 beträgt etwa 1/64, der Korrekturfaktor für die Kurve 20 beträgt etwa 1/11.

Anstelle der Normierung ist es ausreichend, die Kurve 20 so zu transformieren, dass die Amplitude bei 465 nm auf die Amplitude der Kurve 19 abgebildet wird und den so ermittelten Korrekturfaktor, der hier etwa 64/11 beträgt, auf die übrigen Amplituden der Kurve 20 anzuwenden.

Aufgrund der Korrektur erhöht sich das Verhältnis V der Fluoreszenzintensitäten der Kurven 19', 20' bei 675 nm von ursprünglich 3,5 auf 21,4, also auf etwa das Sechsfache. Wäre in Fig. 2 die Lichtübertragung von Lichtleitkabel und Handstück beispielsweise durch Alterung auf die Hälfte gesunken, so würden, da dieser Weg vom Licht zweimal passiert wird, alle Intensitäten auf ein Viertel der dort gezeigten Werte zurückgehen. Somit fiele die Fluoreszenz des Konkrementes bei 675nm unter die Amplitude der sauberen Zahnoberfläche. Da das Gerät auf die optischen Eigenschaften des ungealterten Handstückes eingestellt ist, würde es in diesem Fall das Konkrement nicht als solches anzeigen.

Wesentlich für das Verständnis der Erfindung ist, dass die aus einer oder einer Vielzahl von Messungen an einem gesunden Gewebe gewonnene Kurve 19 bzw. die normierte Kurve 19' oder ein Teil davon als Standardkurve oder als Standardwert für das Gerät vorgegeben ist, dass bei einer Messung also nur die Kurve 20, bzw. Teile der Kurve, gemessen wird und dass die gemessenen Kurve 20, bzw. Teile der Kurve, nach der Normierung oder Transformation unter Verwendung eines Korrekturwertes als Kurve 20', bzw. als Teil der Kurve 20', mit der gespeicherten Kurve 19' bzw. Teilen der Kurve 19' verglichen wird.

Dabei kann beispielsweise aus dem Verhältnis des normierten Werts der Kurve 20' zum Standardwert der Kurve 19' im Bereich einer bestimmten Wellenlänge ein Vergleichswert bestimmt werden, anhand dessen das Überschreiten eines Schwellenwerts überprüft wird. Beträgt der Quotient von normierter gemessener Strahlung zu der normierten erwarteten Strahlung des gesunden Gewebes beispielsweise mehr als 10, so kann von einer krankhaften Veränderung ausgegangen werden. Der Schwellenwert könnte daher auf 10 festgesetzt werden. Im vorliegenden Fall beträgt das Verhältnis V sogar 21,4.

Es ist nicht erforderlich, das in den Kurven 19, 20 dargestellte gesamte Spektrum zu erfassen, vielmehr ist es ausreichend, wenn nur bei bestimmten, aussagefähigen Wellenlängen oder Bereichen von Wellenlängen eine Erfassung und Auswertung erfolgt.

Zusätzlich zu der Auswertung der Strahlung im Fluoreszenzbereich des krankhaften Gewebes kann eine weitere Kontrollauswertung durch Vergleich der Fluoreszenzstrahlung außerhalb des Fluoreszenzbereichs des krankhaften Gewebes und außerhalb des Bereichs der Anregungsstrahlung erfolgen, im vorliegenden Fall beispielsweise bei 520 nm. Dort ist bei einem gesunden Gewebe eine starke Strahlungsintensität der Fluoreszenzstrahlung zu erwarten, bei einem kranken Gewebe hingegen eine vergleichsweise schwache Strahlungsintensität. Aufgrund der Normierung lässt sich aus dem Verhältnis von Intensität des Spektrums des gesunden Bereichs zu Intensität des Spektrums des kranken Bereichs ein Quotient bilden, der ebenfalls einer Schwellwertprüfung unterzogen werden kann.

In der Praxis haben Filter, Empfänger und Strahlungs- bzw. Lichtquellen eine gewisse Bandbreite der abgestrahlten Strahlung, so dass Strahlungsmaxima und mehr oder weniger breite Ränder vorliegen. Zur Verwirklichung der Erfindung ist es ausreichend, wenn anstelle einzelner Wellenlängen Spektren verwendet werden, solange diese gegeneinander abgrenzbar sind.

Fig. 4 zeigt die Einspeisung und die Auskopplung des Lichtes in das Lichtleitkabel in einer beispielhaften Ausführungsform bei zwei Empfangskanälen. Das aus der Lichtquelle 1 austretende Licht wird durch eine Linse 2 auf einen Lichtleiter 4 fokussiert. Zwischen Linse 2 und Lichtleiter 4 befindet sich gegebenenfalls ein Filter 3, welches einen geeigneten Bereich von Wellenlängen des von der Lichtquelle 1 ausgehenden Lichtes durchläßt.

Das zweite Ende des Lichtleiters 4 ist mit einer optischen Steckverbindung 5 verbunden. Dort wird das zum Handstück führende Lichtleitkabel angeschlossen.

Gleichzeitig führt ein zweiter Lichtleiter 7 an der Steckverbindung 5 ankommendes Licht von der Steckverbindung 5 zu einem ersten optischen Empfänger 6. Dieser wandelt das Licht in ein elektrisches Signal um.

Optional kann dem Empfänger 6 ein Filter vorgeschaltet werden, um das relativ starke reflektierte Anregungslicht abzuschwächen. Der optische Empfänger 6 liefert somit das Signal für die beschriebene Normierung.

Ein dritter Lichtleiter 10 führt zu einem zweiten optischen Empfänger 8. Diesem ist ein optisches Filter 9 vorgeschaltet, welches den für eine Auswertung der Fluoreszenz vorgesehenen Spektralbereich des Lichtes auswählt.

In der praktischen Anwendung werden in dem zweiten Empfänger 8 Strahlungen in einem diagnostisch auswertbaren Bereich erfasst. Dieser Bereich umfasst im Falle der Fluoreszenzstrahlung bei Anregung mit Licht zur Erkennung von Konkrementen Wellenlängen zwischen 620 bis 670 nm mit einem Maximum bei etwa 630 nm und zur Erkennung von Karies Wellenlängen zwischen 670 bis 700 nm mit einem Maximum bei etwa 680 nm.

In dem ersten Empfänger 6 wird eine Strahlung außerhalb des diagnostisch auswertbaren Bereichs erfasst, nämlich die zurückgestrahlte Anregungsstrahlung. Bei Anregung mit Licht in einem Wellenlängenbereich von 400 bis 470 nm wird eine gute Fluoreszenzstrahlung am Zahn hervorgerufen, wobei das Maximum der Fluoreszenzstrahlung bei einer Anregung mit Licht mit einer Wellenlänge von etwa 406 nm liegt. Wesentlich ist, dass sich die Wellenlängen der Anregungsstrahlung und der diagnostisch ausgewerteten Fluoreszenzstrahlung nicht überschneiden bzw. dass eventuell vorhandene Bereiche der Überschneidung eine Trennung der Signale gleichwohl zulassen.

Der mit Licht beaufschlagte Empfänger liefert für alles einfallende Licht ein Summensignal. Sollen nur schmalbandige Bereiche ausgewertet werden, so sind entweder entsprechend schmalbandige Empfänger einzusetzen oder aber geeignete Filter vorzusehen.

Eine zweite Ausführungsform zeigt Fig. 5. Das von einer blauen Leuchtdiode 11 ausgehende Licht wird von einem Blau reflektierenden, unter 45 Grad gestellten Farbteilerspiegel 13 auf eine Linse 12 gelenkt und auf den optischen Steckverbinder 5 fokussiert. Dies ist die Anregungsstrahlung.

Das in den Steckverbinder 5 eintreffende Licht, das aus rückgestrahlter Anregungsstrahlung und Fluoreszenzstrahlung besteht, gelangt wiederum über die Linse 12 auf den Farbteilerspiegel 13. Dieser lässt Licht oberhalb einer bestimmten Wellenlänge möglichst ungehindert durch. Gleichzeitig ist er zu einem geringen Teil für das blaue Anregungslicht durchlässig.

Das durch den Farbteilerspiegel (13) hindurchgetretene Licht trifft auf einen weiteren Teilerspiegel (14). Dieser kann wiederum als Farbteiler ausgeführt sein, alternativ teilt er das eintreffende Licht in einem bestimmten Verhältnis ohne Berücksichtigung der Wellenlänge. Von dem Farbteilerspiegel 14 umgelenktes Licht wird von einem Empfänger 8 erfasst, das durch den Farbteilerspiegel 14 hindurchgetretene Licht wird von dem Empfänger 6 erfasst. Die Empfänger 6, 8 arbeiten in einem unterschiedlichen Bereich der Wellenlänge. So erfasst der Empfänger 8 die Strahlung in einem diagnostisch auswertbaren Wellenlängenbereich, der Empfänger 6 erfasst einen Teil der Reflexionsstrahlung mit einer Wellenlänge im Bereich der Anregungsstrahlung.

Die einzelnen Bauteile sind in ein gemeinsames Gehäuse 15 eingebaut, welches darüber hinaus noch nicht dargestellte elektrische Anschlüsse aufweist.

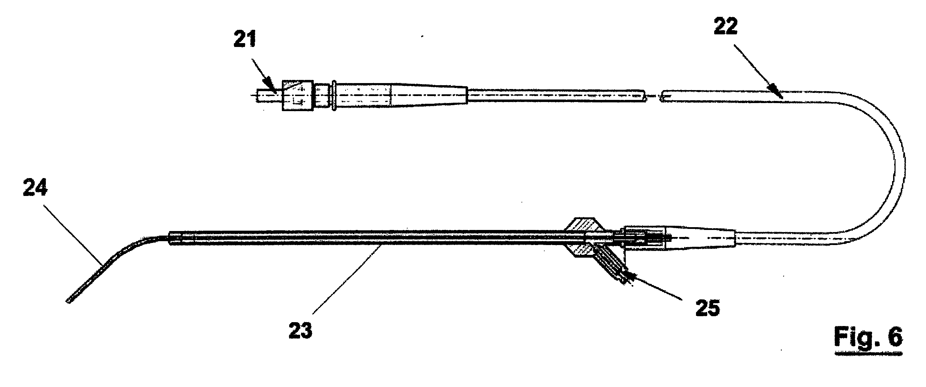

Fig. 6 zeigt beispielhaft ein erfindungsgemäßes Handstück mit Lichtleiter zum Anschluss an die optische Steckverbindung aus Fig. 4 und 5. Es wird mit dem lösbaren optischen Stecker 21 mit der Steckverbindung verbunden. Das Lichtleitkabel 22 transportiert das Licht zum und vom Handstück 23. Das Handstück 23 weist ein distales Ende 24 geringen Querschnittes auf, mit welchem auch enge Partien und insbesondere parodontale Taschen erreichbar sind. Über einen Spülanschluß 25 kann Flüssigkeit durch das Handstück 23 und das distale Ende 24 in die parodontale Tasche gepumpt werden, um dort beispielsweise vorhandenes Blut auszuspülen oder bakterizide Substanzen einzubringen.

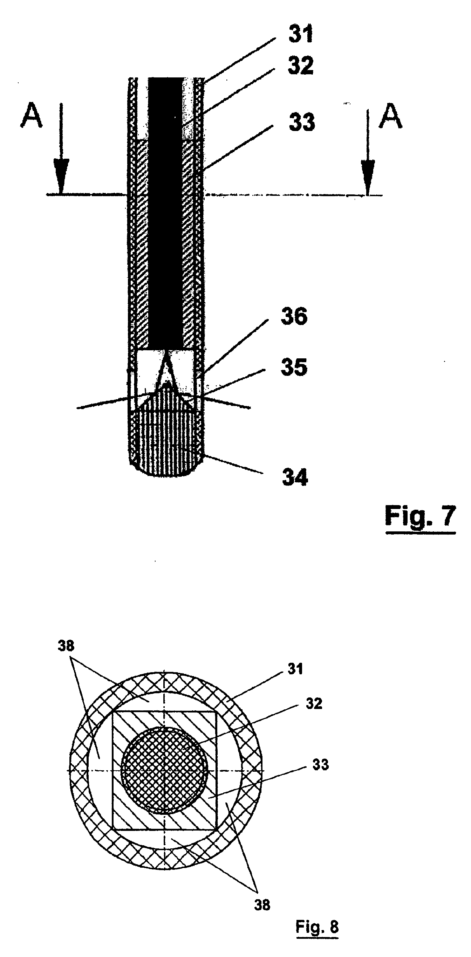

Fig. 7 zeigt eine Ausführungsform des distalen Endes 24 des Handstücks aus Fig. 6. Es ist mit kreisförmigem Querschnitt aufgebaut. Der Außendurchmesser beträgt etwa 1 mm. Im Zentrum eines Rohres 31 ist ein Lichtleiter 32 mit geringerem Außendurchmesser als der Innendurchmesser des Rohrs 31 angebracht. Der Lichtleiter 32 ist abschnittsweise in einem Klemmteil 33 geführt und endet kurz vor einem Körper 34 mit dachförmiger Oberfläche 35. Die dachförmige Oberfläche 35 wirft das Licht in zwei Richtungen senkrecht zur Mittelachse des distalen Endes 24, wo es durch zwei Fenster 36 in dem Rohr 31 austritt. Der Körper 34 und die dachförmige Oberfläche 35 sind so angebracht, dass dann, wenn das Handstück wie in Fig. 6 gezeigt liegt, das Licht senkrecht in und aus der Zeichenebene strahlt. Aus diesen beiden Richtungen wird gleichzeitig von der Umgebung ausgehendes oder zurückgestrahltes Licht empfangen und in den Lichtleiter 32 eingespeist.

Fig. 8 zeigt einen Schnitt des distalen Endes entlang der Linie A aus Fig. 7. Der Lichtleiter 32 wird von dem Klemmteil 33 innerhalb des Rohrs 31 so gehalten, dass zwischen dem Lichtleiter 32 und dem Rohr 31 Öffnungen 38 verbleiben, welche eine Durchfluss der Spülflüssigkeit ermöglichen.

Im Gegensatz zu den bekannten Vorrichtungen wird nicht nur die Fluoreszenz des Gewebes ausgewertet. Vielmehr wird zusätzlich das von dort zurückgestreute (reflektierte) Licht in die Auswertung mit einbezogen. Da die Intensität des reflektierten Lichtes meist sehr viel höher ist als die der Fluoreszenz ist sie gegebenenfalls durch geeignete Maßnahmen im Gerät zu reduzieren.

## Patentansprüche

1. Verfahren zum Erkennen von krankhaften Veränderungen wie Karies, Plaque, Konkrementen oder bakteriellem Befall an einer Gewebeoberfläche, insbesondere an Zähnen, umfassend die Schritte
a) Bestrahlen einer zu untersuchenden Gewebeoberfläche mit einer Anregungsstrahlung mit einem ersten Strahlungsspektrum, die an der Gewebeoberfläche (16) eine Fluoreszenzstrahlung mit einem zweiten Strahlungsspektrum hervorruft, und
b) Erfassen und Auswerten des Strahlungsspektrums der an der Gewebeoberfläche (16) durch die Anregungsstrahlung hervorgerufenen Fluoreszenzstrahlung zumindest in einem Teilbereich mit einer im Falle des Auftretens einer krankhaften Veränderung wie Karies, Plaque, Konkremente oder bakterieller Befall gegenüber der gesunden Gewebeoberfläche erhöhter Fluoreszenzstrahlung,
**dadurch gekennzeichnet, dass**
c) zusätzlich eine Erfassung und Auswertung der von der Gewebeoberfläche (16) rückgestreuten Strahlung in mindestens einem Teilbereich des Strahlungsspektrums der Anregungsstrahlung erfolgt,
d) wobei der Teilbereich der ausgewerteten rückgestreuten Anregungsstrahlung und der Teilbereich der ausgewerteten Fluoreszenzstrahlung zumindest bezüglich der zu erwartenden Maximalwerte überschneidungsfrei sind.

2. Verfahren nach Anspruch 1, **dadurch gekennzeichnet**,
a) dass nach der Auswertung des von der Gewebeoberfläche (16) rückgestreuten Spektrums der Anregungsstrahlung oder eines Teilbereichs davon eine Anpassung (Normierung) des an der Gewebeoberfläche (16) durch die Anregungsstrahlung hervorgerufenen Spektrums der Fluoreszenzstrahlung erfolgt,
b) indem aus dem Spektrum der ausgewerteten rückgestreuten Anregungsstrahlung ein Abbildungsfaktor erzeugt wird, der auf das Spektrum der Fluoreszenzstrahlung angewandt wird,
c) und indem vorzugsweise in einem weiteren Schritt der mit dem Abbildungsfaktor abgebildete Wert des Spektrums der Fluoreszenzstrahlung mit einem vorgegebenen Standardwert verglichen wird.

3. Verfahren nach Anspruch 1 oder 2, **dadurch gekennzeichnet, dass** die Spektren jeweils als schmalbandiger Bereich ausgewählt sind.

4. Verfahren nach einem der Ansprüche 1 bis 3, **dadurch gekennzeichnet, dass** zumindest ein Teil des reflektierten Teils der Anregungsstrahlung in einem ersten Empfänger (6) ausgewertet wird und dass die vom Zahn ausgehende Strahlung vor dem Auftreffen auf einen zweiten Empfänger (8) so gefiltert wird, dass im wesentlichen nur die Fluoreszenzstrahlung und nicht die Anregungsstrahlung erfasst wird.

5. Verfahren nach einem der Ansprüche 1 bis 4, **dadurch gekennzeichnet, dass** als Strahlung Licht und als Messgröße die Lichtintensität zu dem jeweiligen Bereich der Wellenlänge verwendet wird.

6. Vorrichtung zum Erkennen von krankhaften Veränderungen wie Karies, Plaque, Konkrementen oder bakteriellem Befall an einer Gewebeoberfläche, insbesondere an Zähnen, mit einer Einrichtung zur Erzeugung einer Anregungsstrahlung, welche mit Hilfe eines zahnärztlichen Handstücks (23) auf eine zu untersuchende Gewebeoberfläche (16) gerichtet ist, und mit Mitteln zur Übertragung von aus einer Quelle (3) austretender Anregungsstrahlung zu der zu bestrahlenden Gewebeoberfläche und Mitteln (8) zum Erfassen einer an der bestrahlten Gewebeoberfläche angeregten Fluoreszenzstrahlung, **dadurch gekennzeichnet, dass** weiterhin Mittel (6) zum Erfassen einer an der bestrahlten Gewebeoberfläche (16) mit der Wellenlänge der Anregungsstrahlung rückgestreuten Reflexionsstrahlung vorgesehen sind.

7. Vorrichtung nach Anspruch 6, **dadurch gekennzeichnet, dass** die Anregung der zu untersuchenden Oberfläche mit Strahlung eines ersten Bereiches des Strahlungsspektrums erfolgt, dass über Mittel (6, 8) die Erfassung der von der Gewebeoberfläche (16) ausgehenden Strahlung in mindestens zwei überschneidungsfreien Teilbereichen des Strahlungsspektrums erfolgt, wobei einer der Teilbereiche für die Auswertung der Teilbereich der Anregungsstrahlung oder ein weiter eingeschränkter Teilbereich hiervon ist.

8. Vorrichtung nach Anspruch 6 oder 7, **dadurch gekennzeichnet, dass** im Strahlengang der Anregungsstrahlung ein Filter (3) vorgesehen ist, welches nur einen geeigneten Teilbereich der von der Quelle (1) ausgehenden Strahlung durchlässt.

9. Vorrichtung nach einem der Ansprüche 6 bis 8, **dadurch gekennzeichnet, dass** im Strahlengang der Reflexionsstrahlung ein Filter (14) vorgesehen ist, um die relativ stark rückgestreute Anregungsstrahlung abzuschwächen und/oder auf einen Teilbereich zu beschränken.

10. Vorrichtung nach einem der Ansprüche 6 bis 9, **dadurch gekennzeichnet, dass** im Strahlengang der Fluoreszenzstrahlung ein Filter (9, 14) vorgesehen ist, um diese auf einen Teilbereich zu beschränken.

11. Vorrichtung nach einem der Ansprüche 6 bis 10, **dadurch gekennzeichnet, dass** ein Lichtleiter (7) zur Auskopplung von Reflexionsstrahlung zu einem ersten optischen Empfänger (6), der das Licht in ein elektrisches Signal umwandelt, und ein weiterer Lichtleiter (10), der zu einem zweiten optischen Empfänger (8) für eine Auswertung der Fluoreszenzstrahlung innerhalb eines vorgesehenen Spektralbereichs führt, vorgesehen ist.

12. Vorrichtung nach einem der Ansprüche 6 bis 11, **dadurch gekennzeichnet, dass** Korrekturmittel zur Anpassung (Normierung) des am Zahn durch die Anregungsstrahlung hervorgerufenen Teilbereichs der Fluoreszenzstrahlung unter Verwendung eines durch die Reflexionsstrahlung hervorgerufenen Teilbereichs der Anregungsstrahlung vorgesehen sind.

13. Vorrichtung nach einem der Ansprüche 6 bis 12, **dadurch gekennzeichnet, dass** ein Speicherbereich für einen abgelegten Standardwert eines Teilbereichs der Anregungsstrahlung als Vergleichsgröße vorgesehen ist und dass Vergleichsmittel des Standardwerts mit dem nach der Auswertung des vom Zahn reflektierten Teilbereichs der Anregungsstrahlung vorhanden sind.

14. Vorrichtung nach einem der Ansprüche 6 bis 13, **dadurch gekennzeichnet, dass** die Lichtquelle (1) eine gepulste oder modulierte Leuchtdiode oder Laserdiode ist.

15. Vorrichtung nach Anspruch 14, **dadurch gekennzeichnet, dass** die Lichtquelle (1) gefiltert ist.

16. Vorrichtung nach einem der Ansprüche 6 bis 15, **dadurch gekennzeichnet, dass** das Handstück Strahlung, insbesondere Licht, aus mindestens einer seitlichen Richtung, bevorzugt aus einer oder zwei Richtungen unter etwa 90 Grad zu seiner Längsachse, sendet und empfängt.

17. Vorrichtung nach Anspruch 16, **dadurch gekennzeichnet, dass** die Ablenkung der Strahlung durch Reflexion an einer schräggestellten Oberfläche (35) erfolgt.

18. Vorrichtung nach Anspruch 17, **dadurch gekennzeichnet, dass** die Ablenkung der Strahlung durch Reflexion an zwei entgegengesetzt abstrahlenden Oberflächen (35) erfolgt.

19. Vorrichtung nach einem der Ansprüche 6 bis 18, **dadurch gekennzeichnet, dass** das Mittel zur Übertragung der Strahlung aus einem Lichtleiter (22) aus einer einzelnen Faser besteht, welche für die Hin- und Rückleitung des Lichtes benutzt wird.

20. Vorrichtung nach einem der Ansprüche 6 bis 18, **dadurch gekennzeichnet, dass** das Mittel zur Übertragung der Strahlung aus einem Lichtleiter (22) aus einem Faserbündel besteht.

21. Vorrichtung nach einem der Ansprüche 6 bis 19, **dadurch gekennzeichnet, dass** das Handstück (23) mindestens einen Kanal (38) zur Durchleitung einer Flüssigkeit an die Stelle der Bestrahlung aufweist.

22. Vorrichtung nach einem der Ansprüche 6 bis 21, **dadurch gekennzeichnet, dass** das distale Ende des Handstückes (23) zusätzlich Markierungen enthält, welche die Eindringtiefe in die Tasche anzeigen.

23. Vorrichtung nach einem der Ansprüche 6 bis 22, **dadurch gekennzeichnet, dass** eine bakterizide Flüssigkeit verwendet wird.

24. Vorrichtung nach einem der Ansprüche 6 bis 23, **dadurch gekennzeichnet, dass** eine optische, akustische oder sonstige Anzeige für die Überschreitung eines vorgegebenen Schwellenwertes durch einen aus der gemessenen Strahlung zu der erwarteten Strahlung des gesunden Gewebes bestimmten Vergleichswertes vorgesehen ist.

25. Vorrichtung nach Anspruch 24, **dadurch gekennzeichnet, dass** der Schwellenwert einstellbar ist.
